# EUROPÄISCHE PATENTSCHRIFT

(11) **EP 0 798 287 B1**
(45) Veröffentlichungstag und Bekanntmachung des Hinweises auf die Patenterteilung: **30.08.2000**
(21) Anmeldenummer: 97104485.4
(22) Anmeldetag: 17.03.1997
(51) Int. Cl.: C07C 213/06, C07C 219/06

(54) **Verfahren zur Herstellung von Esterquats**
Process for the preparation of quaternary esters
Procédé de préparation d'esters quaternaires

(30) Priorität: 25.03.1996 DE 19611623
(43) Veröffentlichungstag der Anmeldung: 01.10.1997
(73) Patentinhaber: Cognis Deutschland GmbH, 40589 Düsseldorf (DE)
(72) Erfinder: Trius, Antonio, Dr., Valldoreix Sant Cugat (ES); Pi Subirana, Rafael, Dr., 08400 Granollers (ES); Bigorra Llosas, Joaquim, Dr., Esc. E. 4 1A, 08203 Sabadell (ES)

(56) Entgegenhaltungen:
- DE-A- 4 334 365
- DE-C- 4 308 792
- DE-C- 4 308 794
- DE-C- 4 335 782
- DE-C- 4 409 322

## Beschreibung

### Gebiet der Erfindung

Die Erfindung betrifft ein Verfahren zur Herstellung von kationischen Tensiden vom Esterquat-Typ, bei dem man Triglyceride zusammen mit Fettsäuren und ausgewählten Alkanolaminen in Gegenwart von bestimmten Polyolen partiell umestert und anschließend quaterniert.

### Stand der Technik

Kationische Tenside vom Esterquat-Typ haben wegen ihrer vorteilhaften ökotoxikologischen Eigenschaften in den vergangenen Jahren sowohl als Avivagemittel für Textilfasem als auch als Bestandteil von Haarpflegemitteln weltweit an Bedeutung gewonnen. Übersichten zu diesem Thema sind beispielsweise von O.Ponsati in **C.R. CED-Kongress, Barcelona, 1992, S.167**, R.Puchta et al. in **Tens. Surf.Det., 30, 186 (1993)**, M.Brock in **Tens.Surf.Det. 30, 394 (1993)** und R.Lagerman et al. in **J.Am. Oil.Chem.Soc., 71, 97 (1994)** erschienen. Zur Herstellung von Esterquats geht man üblicherweise von Fettsäuren und mehrwertigen Alkanolaminen aus, die zunächst verestert und dann quaterniert werden, wie dies beispielsweise in der **WO 91/01295** (Henkel) beschrieben wird. Weitere Verfahren zur Herstellung von Esterquat-Tensiden sind aus den Druckschriften **DE-C1 4308792, DE-A1 4334365** und **DE-C1 4409322** (Henkel) bekannt, wobei letzte die Umesterung von Triglyceriden mit Alkanolaminen und Fettsäuren sowie die nachfolgende Quaternierung der resultierenden Ester beschreibt. Die Quaternierung in Gegenwart von Emulgatoren bzw. Dispergatoren, wie z.B. Glycerin, wird beispielsweise in den Schriften **DE-C1 4308794, DE-C1 4335782** und **DE-C1 4339643** (Henkel) beschrieben. Gegenstand der **DE-A1 4215689** (Hüls) ist die Quaternierung von Triethanolaminestern in Gegenwart von propoxylierten Triglyceriden als Lösungsmittel. Obschon theoretisch eine Vielzahl verschiedener Esterquat-Typen denkbar sind, haben sich in der Vergangenheit doch nur vergleichsweise wenige Produkte als anwendungstechnisch erfolgversprechend erwiesen. So kommen als geeignete Ausgangsstoffe überwiegend gesättigte Fettsäuren mit 16 bis 18 Kohlenstoffatomen in Betracht, da Produkte auf Basis kürzerkettigerer und/oder ungesättigter Fettsäuren keine zufriedenstellenden Avivageeigenschaften aufweisen. Der Veresterungsgrad, der bei Einsatz von Triethanolamin als mehrwertigem Alkanolamin ja 1 bis 3 betragen kann, liegt vorzugsweise in der Umgebung von 2, da die Vergangenheit erwiesen hat, daß es zum Aufziehen auf Fasern vorzugsweise zweier Fettreste bedarf. Auch bei der Auswahl des Alkylierungsmittels gibt es keine große Variationsmöglichkeit, kommen doch aus Gründen der Verfügbarkeit eigentlich nur Methylchlorid oder Dimethylsulfat in Betracht. Für den Fachmann bedeutet dies, daß er für das Einstellen eines gewünschten Eigenschaftsprofils seiner Esterquats tatsächlich nur sehr wenige Parameter zur Verfügung hat und sich das Maßschneidem von Produkten im wesentlichen auf die Abmischung mit geeigneten Zusatzstoffen beschränkt.

Es besteht daher seit langem ein Bedürfnis nach einem Verfahren zur Herstellung von Esterquats, das zum einen technisch einfach durchzuführen ist und die Verwendung von wenig veredelten Rohstoffen ermöglicht und zum anderen doch eine Feinregelung der Eigenschaften wie z.B. Weichgriff, Antistatik, Hydrophilie und dergleichen zuläßt. Die Aufgabe der Erfindung hat darin bestanden, ein solches Verfahren zur Verfügung zu stellen.

### Beschreibung der Erfindung

Gegenstand der Erfindung ist ein Verfahren zur Herstellung von Esterquats durch partielle Umesterung von Mischungen aus Triglyceriden und Fettsäuren mit Triethanolamin und/oder Methyldiethanolamin in Gegenwart von Reduktionsmitteln sowie anschließende Quaternierung der Reaktionsprodukte mit Alkylierungsmitteln, welches sich dadurch auszeichnet, daß man die Umesterung in Gegenwart von Polyolen mit 2 bis 15 Kohlenstoffatomen und mindestens 2 Hydroxylgruppen durchführt.

Das Verfahren bietet nicht nur die aus ökonomischer Sicht wichtige Möglichkeit, unmittelbar von Fetten und Ölen auszugehen, überraschenderweise wurde zudem gefunden, daß durch Variation des Umesterungsgrades und des Gehaltes an freier Fettsäure sowie dem Einbau von Polyolen in die Esterstrukturen nach der Quaternierung Produkte mit einem breiten Spektrum an Eigenschaften erhalten werden. Durch Steuerung der bereits genannten Parameter zusammen mit der Auswahl der Einsatzstoffe und des Quaternierungsgrades lassen sich Esterquats mit "maßgeschneiderten" Eigenschaften erhalten.

### Triglyceride

Geeignete Triglyceride stellen neben synthetischen Produkten selbstverständlich in erster Linie technische Fette und Öle dar, die man aus natürlichen Rohstoffen unmittelbar nach der Raffination erhält. Die Triglyceride folgen in Summe der Formel **(I)**, 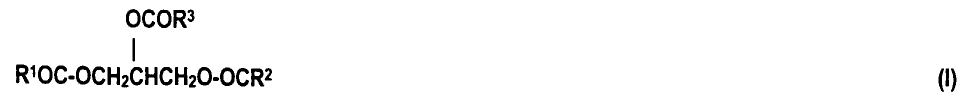 in der R¹CO, R²CO und R³CO unabhängig voneinander für Acylreste mit 6 bis 22 Kohlenstoffatomen stehen. Typische Beispiele für geeignete Fette und Öle sind Palmöl, Palmkemöl, Kokosöl und insbesondere Rindertalg. Vorzugsweise werden die Fette und Öle nach der Härtung eingesetzt, d.h. sie weisen lodzahlen im Bereich von 0 bis 5 auf. Daneben kommen auch teilgehärtete Produkte in Betracht, deren lodzahlen im Vergleich zu den Ausgangsstoffen auf etwa 40 bis 60 % des Ausgangswertes herabgesetzt worden sind und vorzugsweise im Bereich von 40 bis 60 liegen. In diesem Zusammenhang besonders bevorzugt sind teilgehärteter Rindertalg und teilgehärtetes Palmöl.

### Fettsäuren

Unter geeigneten Fettsäuren sind aliphatische Carbonsäuren der Formel **(II)** zu verstehen,

**R**^{**4**}**CO-OH (II)**

in der R⁴CO für einen aliphatischen, linearen oder verzweigten Acylrest mit 6 bis 22 Kohlenstoffatomen und 0 und/oder 1, 2 oder 3 Doppelbindungen steht. Typische Beispiele sind Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen. Bevorzugt sind technische Fettsäuren mit 12 bis 18 Kohlenstoffatomen wie beispielsweise gehärtete oder teilgehärtete Kokos-, Palm-, Palmkern- oder insbesondere Talgfettsäure. Das molare Einsatzverhältnis von Triglyceriden und Fettsäuren kann 50 : 1 bis 10 : 1, vorzugsweise 40 : 1 bis 10 : 1 und insbesondere 30 : 1 bis 20 : 1 betragen.

### Triethanolamin und Methyldiethanolamin

Als geeignete Alkanolamine kommen Triethanolamin und Methyldiethanolamin sowie deren Mischungen im Gewichtsverhältnis 95 : 5 bis 5 : 95 in Betracht. Das molare Einsatzverhältnis von Triethanolamin und/oder Methyldiethanolamin zu den Fettsäuregruppen (also der Summe aus den eingesetzten freien Fettsäuren und den Fettsäuregruppen in den Triglyceriden) kann 40 : 1 bis 1 : 1, vorzugsweise 30 : 1 bis 5 : 1 und insbesondere 25 : 1 bis 10 : 1 betragen.

### Polyole

Als Polyole, die im Sinne der Erfindung einkondensiert werden, kommen solche mit 2 bis 15 Kohlenstoffatomen und mindestens 2 Hydroxylgruppen in Betracht. Typische Beispiele sind
- Glycerin;
- Alkylenglycole wie beispielsweise Ethylenglycol, Diethylenglycol, Propylenglycol;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Üblicherweise setzt man die Triglyceride und die Polyole im molaren Verhältnis von 20 : 1 bis 1 : 1 und vorzugsweise 4 : 1 bis 2 : 1 ein. Ein typisches Beispiel für eine Polyolmischung stellt eine Mischung von Glycerin und Pentaerythrit im molaren Verhältnis 95 : 5 dar.

### Umesterung

Die Umesterung der Mischungen aus Triglyceriden, Fettsäuren, Alkanolaminen und Polyolen kann in an sich bekannter Weise, d.h. bei Temperaturen im Bereich von 180 bis 220 und vorzugsweise 190 bis 200°C durchgeführt werden. Es empfiehlt sich, das Kondensationswasser kontinuierlich aus dem Reaktionsansatz zu entfernen, um so das Gleichgewicht auf die Seite der Zielprodukte zu verlagern. Als Katalysatoren kommen Hypophosphorsäure und deren Alkali- und/oder Erdalkalisalze in Betracht, die in Mengen von 0,01 bis 0,5 Gew.-% - bezogen auf die Einsatzstoffe - eingesetzt werden können. Besonders vorteilhaft ist die Mitverwendung von Reduktionsmitteln, wie insbesondere Natriumborhydrid, in Mengen von 10 bis 50 Gew.-% - bezogen auf den Katalysator - da die Co-Katalysatoren nicht nur die Umesterung unterstützen, sondern auch die Farbqualität der Reaktionsprodukte verbessern. Nicht umgeesterte Triglyceride und nicht abreagierte freie Fettsäuren verbleiben in der Mischung.

### Quaternierung

Auch die Quaternierung kann in an sich bekannter Weise erfolgen, wobei als Alkylierungsmittel in erster Linie Dialkylsulfate, vorzugsweise Dimethylsulfat, oder Alkylhalogenide, vorzugsweise Methylchlorid, zum Einsatz gelangen. Die Alkylierung findet in der Regel bei Temperaturen im Bereich von 50 bis 90 und vorzugsweise 60 bis 70°C statt, wobei sich die Menge an Alkylierungsmittel danach richtet, welches Eigenschaftsprofil eingestellt werden soll. Demzufolge kann die Einsatzmenge zwischen 50 und 105 und vorzugsweise 90 bis 100 Mol-% - bezogen auf den für die Quaternierung zur Verfügung stehenden Stickstoff - liegen. Falls erforderlich, kann nicht umgesetztes Alkylierungsmittel beispielsweise durch Zugabe von Monoethanolamin oder Glycin nachträglich zerstört werden. Der Aktivsubstanzgehalt, d.h. die Menge an kationaktivem Tensid, liegt üblicherweise im Bereich von 0,1 bis 1,0 meq/g. Ein besonderer Vorteil des erfindungsgemäßen Verfahrens besteht darin, daß der als Zwischenprodukt anfallende Ester nicht mehr isoliert und vor der Quaternierung in ein Lösungsmittel eingebracht werden muß. Falls gewünscht, ist dies jedoch möglich, d.h. man kann den Ester in geeigneten Lösungsmitteln, vorzugsweise Isopropylalkohol aufnehmen und dann quaternieren.

Es ist ebenfalls möglich, die Quaternierung in Gegenwart von geeigneten Emulgatoren bzw. Dispergatoren vorzunehmen, deren Auswahl sich nach der späteren Verwendung richtet. Geeignete Emulgatoren sind beispielsweise die schon vorher genannten Polyole, aber insbesondere auch Alkylpolyglucoside, Fettsäure-N-alkylglucamide, Polyglycerinpoly-12-hydroxystearate, Fettalkohole mit 12 bis 22 und insbesondere 16 bis 18 Kohlenstoffatomen, sowie Anlagerungsprodukte von 1 bis 40 und insbesondere 5 bis 20 Mol Ethylenoxid an Fettsäurepartialglyceride oder die oben genannten Fettalkohole. Diese Verfahrensweise ist insbesondere dann von Vorteil, wenn schuppbare und leicht wasserlösliche Esterquats erhalten werden sollen, wie sie insbesondere für die Verwendung in der Haarkosmetik gewünscht werden. Das Gewichtsverhältnis zwischen den zu quaternierenden Estern und den Emulgatoren bzw. Dispergatoren kann üblicherweise 90 : 10 bis 10 : 90 und insbesondere 75 : 25 bis 25 : 75 betragen.

### Gewerbliche Anwendbarkeit

Die nach dem erfindungsgemäßen Verfahren erhältlichen Esterquats weisen ausgezeichnete anwendungstechnische Eigenschaften auf und können daher zur Herstellung von Softenem in der textilen Vorbehandlung, Wäscheweichspülmitteln, Papierhilfsmitteln und Haarpflegeprodukten eingesetzt werden, in denen sie in Mengen von 1 bis 50, vorzugsweise 5 bis 30 Gew.-% enthalten sein können. Zur Herstellung von Produkten für die Faseravivage eignen sich insbesondere solche Esterquats, die eine relativ geringe Restsäurezahl, vorzugsweise von weniger als 2 aufweisen und bei denen 90 bis 100 Mol-% des zur Verfügung stehenden Stickstoffs quaterniert vorliegt. Für die Haaravivage kommen eher solche Produkte in Betracht, die eine Säurezahl im Bereich von 2 bis 5 aufweisen, in wäßriger Lösung einen pH-Wert von < 4 ergeben und bei denen 70 bis 90 % des zur Verfügung stehenden Stickstoffs quaterniert vorliegt.

### Beispiel

In einem 2-I-Dreihalskolben mit Rührer und Destillationsaufsatz wurden 933 (1,1 mol) gehärteter Rindertalg, 11 g (0,04 mol) teilgehärtete Talgfettsäure 60 g (0,4 mol) Triethanolamin, 50 g (0,37 mol) Pentaerythrit, 0,6 g Natriumhypophosphit und 0,2 g Natriumborhydrid bei 85°C vermischt. Die Reaktionsmischung wurde 4 h bei 200°C gerührt und das freiwerdende Kondensationswasser kontinuierlich aus dem Gleichgewicht entfernt. Es wurde ein Ester mit einer Aminzahl von 20 mg KOH/g erhalten. Anschließend wurden 1002 g (0,36 mol) des auf diese Weise hergestellten Esters bei 50°C mit 43 g (0,34 mol) Dimethylsulfat versetzt und 3 h gerührt, wobei die Temperatur bis auf 70°C anstieg. Es wurde eine feste, weiß-gelbliche Masse erhalten (Säurezahl 2,2, Aktivsubstanzgehalt 0,312 meq/g), die sich leicht zu Schuppen verarbeiten ließ.

## Patentansprüche

1. Verfahren zur Herstellung von Esterquats durch partielle Umesterung von Mischungen aus Triglyceriden und Fettsäuren mit Triethanolamin und/oder Methyldiethanolamin in Gegenwart von Reduktionsmitteln sowie anschließende Quaternierung der Reaktionsprodukte mit Alkylierungsmitteln, **dadurch gekennzeichnet**, daß man die Umesterung in Gegenwart von Polyolen mit 2 bis 15 Kohlenstoffatomen und mindestens 2 Hydroxylgruppen durchführt.

2. Verfahren nach Anspruch 1, **dadurch gekennzeichnet**, daß man Triglyceride der Formel **(I)** einsetzt, 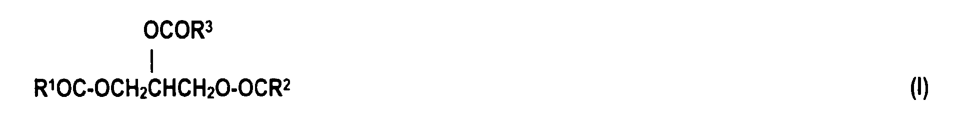 in der R¹CO, R²CO und R³CO unabhängig voneinander für Acylreste mit 6 bis 22 Kohlenstoffatomen stehen.

3. Verfahren nach den Ansprüchen 1 und/oder 2, **dadurch gekennzeichnet**, daß man Fettsäuren der Formel **(II)** einsetzt,
**R**^{**4**}**CO-OH (II)**
in der R⁴CO für einen aliphatischen, linearen oder verzweigten Acylrest mit 6 bis 22 Kohlenstoffatomen und 0 und/oder 1, 2 oder 3 Doppelbindungen steht.

4. Verfahren nach mindestens einem der Ansprüche 1 bis 3, **dadurch gekennzeichnet**, daß man die Triglyceride und die Fettsäuren im molaren Verhältnis von 50 : 1 bis 10 : 1 einsetzt.

5. Verfahren nach mindestens einem der Ansprüche 1 bis 4, **dadurch gekennzeichnet**, daß man die Alkanolamine und die Fettsäuren im molaren Verhältnis von 40 : 1 bis 1 : 1 einsetzt.

6. Verfahren nach mindestens einem der Ansprüche 1 bis 5, **dadurch gekennzeichnet**, daß man Polyole einsetzt, die ausgewählt sind aus der Gruppe, die gebildet wird von Glycerin, Alkylenglycolen und Methylolverbindungen.

7. Verfahren nach mindestens einem der Ansprüche 1 bis 6, **dadurch gekennzeichnet**, daß man die Triglyceride und die Polyole im molaren Verhältnis von 20 : 1 bis 1:1 einsetzt.

8. Verfahren nach mindestens einem der Ansprüche 1 bis 7, **dadurch gekennzeichnet**, daß man die Umesterung bei Temperaturen im Bereich von 180 bis 220°C durchführt und das Kondensationswasser kontinuierlich aus dem Reaktionsgleichgewicht entfernt.

9. Verfahren nach mindestens einem der Ansprüche 1 bis 8, **dadurch gekennzeichnet**, daß man die Quaternierung bei Temperaturen im Bereich von 50 bis 90°C mit Dimethylsulfat oder Methylchlorid durchführt.

## Claims

1. A process for the production of esterquats by partial transesterification of mixtures of triglycerides and fatty acids with triethanolamine and/or methyl diethanolamine in the presence of reducing agents and subsequent quaternization of the reaction products with alkylating agents, characterized in that the transesterification is carried out in the presence of polyols containing 2 to 15 carbon atoms and at least 2 hydroxyl groups.

2. A process as claimed in claim 1, characterized in that triglycerides corresponding to formula (I): 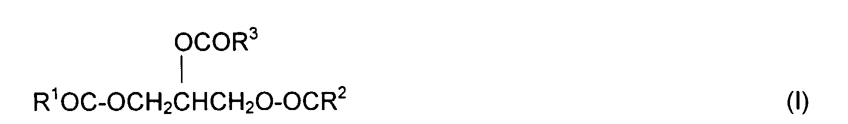 in which R¹CO, R²CO and R³CO independently of one another represent acyl groups containing 6 to 22 carbon atoms,
are used.

3. A process as claimed in claims 1 and/or 2, characterized in that fatty acids corresponding to formula (II):
R⁴CO-OH (II)
in which R⁴CO is an aliphatic, linear or branched acyl group containing 6 to 22 carbon atoms and 0 and/or 1, 2 or 3 double bonds,
are used.

4. A process as claimed in at least one of claims 1 to 3, characterized in that the triglycerides and the fatty acids are used in a molar ratio of 50:1 to 10:1.

5. A process as claimed in at least one of claims 1 to 4, characterized in that the alkanolamines and the fatty acids are used in a molar ratio of 40:1 to 1:1.

6. A process as claimed in at least one of claims 1 to 5, characterized in that polyols selected from the group consisting of glycerol, alkylene glycols and methylol compounds are used.

7. A process as claimed in at least one of claims 1 to 6, characterized in that the triglycerides and the polyols are used in a molar ratio of 20:1 to 1:1.

8. A process as claimed in at least one of claims 1 to 7, characterized in that the transesterification is carried out at temperatures of 180 to 220°C and the water of condensation is continuously removed from the reaction equilibrium.

9. A process as claimed in at least one of claims 1 to 8, characterized in that the quaternization is carried out with dimethyl sulfate or methyl chloride at temperatures of 50 to 90°C.

## Revendications

1. Procédé de production d'esters quaternaires par transestérification partielle de mélanges de triglycérides et d'acides gras avec de la triéthanolamine et/ou de la méthyldiéthanolamine en présence de réducteurs ainsi que par quaternisation ultérieure des produits de la réaction avec des agents alkylants,
caractérisé en ce qu'
on conduit la transestérification en présence de polyols ayant de 2 à 15 atomes de carbone et au moins deux groupes hydroxyles.

2. Procédé selon la revendication 1,
caractérisé en ce qu'
on utilise des triglycérides de formule (I), 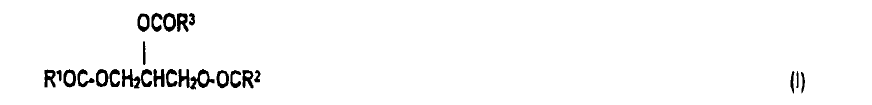 dans laquelle R¹CO, R²CO et R³CO représentent indépendamment l'un de l'autre des radicaux acyles ayant de 6 à 22 atomes de carbone.

3. Procédé selon les revendications 1 et/ou 2,
caractérisé en ce qu'
on utilise des acides gras de formule (II),
**R**^{**4**}**CO-OH (II)**
dans laquelle R⁴CO représente un radical acyle aliphatique, linéaire ou ramifié ayant de 6 à 22 atomes de carbone et comportant 0 et/ou 1, 2 ou 3 doubles liaisons.

4. Procédé selon au moins une des revendications 1 à 3, caractérisé en ce qu'
on utilise les triglycérides et les acides gras dans un rapport molaire de 50:1 à 10:1.

5. Procédé selon au moins une des revendications 1 à 4,
caractérisé en ce qu'
on utilise les alcanolamines et les acides gras dans un rapport molaire de 40:1 à 11.

6. Procédé selon au moins une des revendications 1 à 5,
caractérisé en ce qu'
on utilise des polyols qui sont choisis dans le groupe formé par la glycérine, les alkylènes glycols et les composés méthylols.

7. Procédé selon au moins une des revendications 1 à 5,
caractérisé en ce qu'
on utilise les triglycérides et les polyols dans un rapport molaire de 20:1 à 1:1.

8. Procédé selon au moins une des revendications 1 à 7,
caractérisé en ce qu'
on conduit la transestérification à des températures comprises entre 180 et 220°C, et on recueille de façon continue l'eau de réaction de l'équilibre de cette réaction.

9. Procédé selon au moins une des revendications 1 à 8,
caractérisé en ce qu'
on conduit la quaternisation à des températures comprises entre 50 et 90°C avec du sulfate de diméthyle ou du chlorure de méthyle.
